# EUROPÄISCHE PATENTANMELDUNG

(11) **EP 0 537 605 A2**
(43) Veröffentlichungstag der Anmeldung: **21.04.1993**
(21) Anmeldenummer: 92117084.1
(22) Anmeldetag: 07.10.1992
(51) Int. Cl.: C07C 33/46, C07C 29/145, C07C 29/141

(54) **Verfahren zur Herstellung von 4-Halogenbenzylalkoholen**

(30) Priorität: 18.10.1991 DE 4134497
(71) Anmelder: BASF Aktiengesellschaft, D-67063 Ludwigshafen (DE)
(72) Erfinder: Merger, Franz, Dr., W-6710 Frankenthal (DE); Brudermueller, Martin, Dr., W-6800 Mannheim 1 (DE); Schmidt-Radde, Martin, Dr., W-6700 Ludwigshafen (DE)

(57) **Zusammenfassung**

Verfahren zur Herstellung von 4-Halogenbenzylalkoholen der allgemeinen Formel I 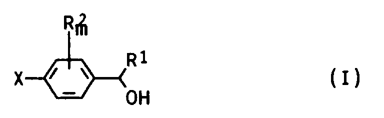
in der
- R¹: Wasserstoff, C₁- bis C₂₀-Alkyl, C₂- bis C₂₀-Alkoxyalkyl, C₃- bis C₂₀-Cycloalkyl, C₄- bis C₃₀-Cycloalkyl-alkyl,
- R²: C₁- bis C₂₀-Alkyl, C₂- bis C₂₀-Alkoxyalkyl, C₃- bis C₂₀-Cycloalkyl, C₄- bis C₃₀-Cycloalkyl-alkyl,
- X: Halogen und
- m: 0 bis 2
bedeuten, indem man halogenaromatische Carbonylverbindungen der Formel II 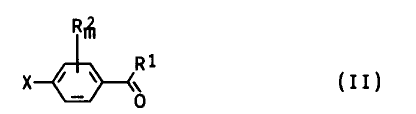
in der die Substituenten R¹, R² und X und der Index m die oben genannte Bedeutung haben, in Gegenwart eines Amins an Kupferkatalysatoren gegebenenfalls in Gegenwart eines inerten Lösungsmittels bei 50 bis 130°C und Drücken von 10 bis 200 bar, mit Wasserstoff umsetzt.

## Beschreibung

Die vorliegende Erfindung betrifft ein Verfahren zur Herstellung von 4-Halogenbenzylalkoholen durch katalytische Hydrierung von halogenaromatischen Carbonylverbindungen in Gegenwart von Kupferoxidkatalysatoren.

Die katalytische Hydrierung von halogenaromatischen Carbonylverbindungen in Gegenwart von Kupferchromitkatalysatoren ist aus DE-A-16 43 876 und US-A-3,527,817 bekannt. Ortho-Chlorphenylketone bzw. Gemische mit seinen meta- und para-chlorierten Isomeren oder mit dichlorierten Verbindungen können danach an Kupferchromitkatalysatoren bei gegenüber halogenfreien Verbindungen erhöhter Temperatur nur dann mit ausreichender Hydriergeschwindigkeit zu den entsprechenden Carbinolen hydriert werden, wenn in Gegenwart von Erdalkalimetallhydroxiden gearbeitet wird. Die alleinige Anwesenheit von anderen basischen Verbindungen hat danach keinen Einfluß auf die Hydrierung bzw. wirkt inhibierend. Lediglich der Zusatz von Aminen mit einem pKa von mindestens 3,7 zusätzlich zu den Erdalkalimetallhydroxiden wirkt bei der Hydrierung von ortho-Chlorphenylketonen beschleunigend.

Das in DE-A-16 43 876 beschriebene Verfahren weist folgende Nachteile auf:
- Die Hydrierung erfolgt bei gegenüber halogenfreien Carbonylverbindungen erhöhten Temperaturen von 100 bis 175°C, vorzugsweise über 150°C.
- Der Zusatz von Aminen ist nur in Verbindung mit Erdalkalihydroxiden möglich.
- Die Katalysatoren sind chromhaltig.
- Der Einsatz von Erdalkalihydroxid bringt für die kontinuierliche Hydrierung erhebliche Schwierigkeiten. Ein Verfahren dieser Art ist technisch nicht realisierbar, da die Erdalkalihydroxide im Reaktionsmedium schwerlöslich bzw. unlöslich sind. Ferner führen Ablagerungen zu rascher Desaktivierung der Katalysatoren (geringe Standzeit).

Der vorliegenden Erfindung lag daher die Aufgabe zugrunde, den zuvor genannten Nachteilen abzuhelfen.

Demgemäß wurde ein neues und verbessertes Verfahren zur Herstellung von 4-Halogenbenzylalkoholen der allgemeinen Formel I 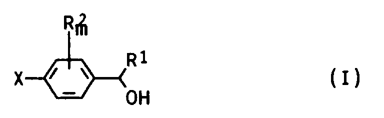
in der
- R¹: Wasserstoff, C₁- bis C₂₀-Alkyl, C₂- bis C₂₀-Alkoxyalkyl, C₃- bis C₂₀-Cycloalkyl, C₄- bis C₃₀-Cycloalkyl-alkyl,
- R²: C₁- bis C₂₀-Alkyl, C₂- bis C₂₀-Alkoxyalkyl, C₃- bis C₂₀-Cycloalkyl, C₄- bis C₃₀-Cycloalkyl-alkyl,
- X: Halogen und
- m: 0 bis 2
bedeuten, welches dadurch gekennzeichnet ist, daß man halogenaromatische Carbonylverbindungen der Formel II 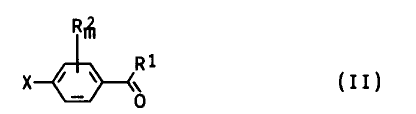
in der die Substituenten R¹, R² und X und der Index m die oben genannte Bedeutung haben, in Gegenwart eines Amins an Kupferkatalysatoren gegebenenfalls in Gegenwart eines inerten Lösungsmittels bei 50 bis 130°C und Drücken von 10 bis 200 bar, mit Wasserstoff umsetzt.

Das erfindungsgemäße Verfahren kann wie folgt durchgeführt werden:
Die Umsetzung kann durch Vorlegen der halogenaromatischen Carbonylverbindungen II in Gegenwart eines Amins, gegebenenfalls in Gegenwart eines inerten Lösungsmittels, an einem Kupferkatalysator mit Wasserstoff bei Temperaturen Von 50 bis 130°C, bevorzugt 70 bis 100°C unter Eigendruck oder durch Aufpressen d.h. bei Drücken von 10 bis 200 bar, bevorzugt bei 50 bis 150 bar durchgeführt werden.

Die Hydrierung kann in der Flüssigphase oder Gasphase diskontinuierlich oder vorzugsweise kontinuierlich, wahlweise in Sumpf- oder Rieselfahrweise in Rohr- oder Rührreaktoren an Katalysatoren in Suspension, vorzugsweise im Festbett mit einer Katalysatorbelastung von 0,05 bis 1 kg Einsatzgemisch/(kg Katalysator h), vorzugsweise mit 0,05 bis 0,3 kg Einsatzgemisch/(kg Katalysator h) erfolgen.

Als Kupferkatalysatoren eignen sich Katalysatoren, deren katalytisch aktive Masse zu 70 bis 100 Gew.%, bevorzugt zu 85 bis 100 Gew.%, besonders zu 95 bis 100 Gew.% aus Kupferoxid besteht und die im Wesentlichen chromoxidfrei sind. Die Kupferoxidkatalysatoren können 0 bis 30 Gew.%, bevorzugt 0 bis 15 Gew.%, besonders bevorzugt 0 bis 5 Gew.%, als katalytisch aktive Masse enthalten. Als Kupferoxide eignen sich sowohl Kupfer-I-oxid, als auch Kupfer-II-oxid und deren Gemische. Die Kupferkatalysatoren können als Vollkatalysatoren oder bevorzugt auf inerten Trägermaterialien, wie Aluminiumoxid, Kieselsäure, Titanoxid, Zirkonoxid oder Magnesiumsilikat oder Oxiden der Elemente der dritten und vierten Hauptgruppe eingesetzt werden.

Die Einsatzmischung kann beispielsweise 40 bis 80 Gew.% einer halogenaromatische Carbonylverbindung II, 15 bis 55 Gew.% eines inerten Lösungsmittels und 1 bis 10 Gew.% eines Amins enthalten. Die zugesetzte Menge Amin sollte in der Regel nicht weniger als 1 Gew.% bezogen auf die halogenaromatische Carbonylverbindung II, also zwischen 1 und 10 Gew.%, bevorzugt 1,5 bis 5 Gew.% betragen.

Die halogenaromatische Carbonylverbindung II kann vorzugsweise in einem inerten Lösungsmittel gelöst zur Reaktion gebracht werden.

Als inerte Lösungsmittel eignen sich Ether wie z.B. Tetrahydrofuran, Dioxan, Dibutylether, Dipropylether und Diethylether, inerte Kohlenwasserstoffe wie Toluol und Xylol, bevorzugt Alkohole wie C₁- bis C₂₀-Alkanole, bevorzugt C₁- bis C₈-Alkanole wie Methanol Ethanol, n-Propanol, i-Propanol und n-Butanol, besonders bevorzugt Ethanol.

Als Amine eignen sich primäre, sekundäre und tertiäre acyclische, cyclische und heterocyclische Amine wie C₁- bis C₂₀-Alkylamine wie Methyl-, Dimethyl-, Trimethylamin, Ethyl-, Diethyl-, Triethylamin, Propyl-, Dipropyl-, Tripropyl-, Butyl-, Dibutyl-, Tributyl-, Pentyl-, Dipentyl-, Hexyl-, Heptyl-, Octyl-, Nonyl-, Decyl-, Dodecylamin, bevorzugt Triethylamin, Diethylamin, Hexadecanamin, Octadecanamin, C₁- bis C₂₀-Monoalkanolamine wie z.B. Dimethyl(hydroxyethyl)-, Methyl(hydroxyethyl)-, C₂- bis C₂₀-Dialkanolamine wie z.B. Methyl-bis(hydroxyethyl)-, sowie heterocyclische Amine wie beispeilsweise Pyridin, Morpholin, Chinolin, Piperidin, Piperazin bzw. Derivate von diesen mit ein bis fünf C₁- bis C₈-Alkylgruppen, bevorzugt mit ein bis drei C₁- bis C₄-Alkylgruppen wie Methyl, Ethyl, n-Propyl, iso-Propyl, n-Butyl, iso-Butyl, sec.-Butyl und tert.-Butyl. Es können auch Gemische der verschiedenen zuvorgenannten Amine eingesetzt werden.

Das erfindungsgemäße Verfahren zur Hydrierung von halogenaromatischen Carbonylverbindungen verläuft an chromfreien Kupferoxidkatalysatoren unter erheblich milderen Reaktionsbedingungen. Ferner verläuft die Hydrierung unter Zusatz eines Amins als Base ohne Verwendung von festen, technisch unpraktikablen Reaktionszusätzen wie anorganischen Basen z.B. Alkali- bzw. Erdalkalihydroxiden, die zu heterogenen Reaktionsgemischen führen.

Bei der erfindungsgemäßen Hydrierung z.B. von p-Chloracetophenon unter Druck in Gegenwart von 2 bis 10 Gew.% eines Amins und 15 bis 55 Gew.% Ethanol als Lösungsmittel, war nach über 900 Betriebsstunden keine Desaktivierung der Kupferoxidkatalysatoren eingetreten.

Durch destillative Auftrennung können die Reaktionsprodukte von zugesetztem Amin und dem Lösungsmittel abgetrennt werden und sowohl das Amin als auch das Lösungsmittel können wieder in die Hydrierung zurückgeführt werden.

Die halogenaromatischen Carbonylverbindungen II sind aus Houben-Weyl, Methoden der organischen Chemie, Bd. 7/2a, S. 39ff bekannt, und können nach darin beschriebenen Verfahren hergestellt werden.

Die Substituenten R¹, R² und X sowie der Index m haben folgende Bedeutungen:
- R¹, R²: - unabhängig voneinander
- C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₂- bis C₂₀-Alkoxyalkyl, bevorzugt C₂- bis C₈-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxy-ethyl,
- C₁- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₃₀-Cycloalkyl-alkyl, bevorzugt C₄- bis C₂₀-Cycloalkyl-alkyl wie Cyclopentyl-methyl,
- R¹: - zusätzlich Wasserstoff,
- X: - Halogen wie Fluor, Chlor Brom und Jod, bevorzugt Chlor und Brom, besonders bevorzugt Chlor,
- m: - eine ganze Zahl von 0 bis 2 wie 0, 1 und 2, bevorzugt 0 oder 1,
- R³,R⁴,R⁵: - Wasserstoff,
- C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
wobei mindestens einer der Reste R³, R⁴, R⁵ ungleich Wasserstoff ist.

Die 4-Halogenbenzylalkohole I sind Zwischenverbindungen zur Herstellung von substituierten Styrolen (US-A-3,927,133, Org. Synthesis Collective Volume 3, 204-206).

### Beispiele

### Beispiel 1

Eine Lösung aus 65 Gew.% 4-Chloracetophenon, 30 Gew.% Ethanol und 5 Gew.% Triethylamin wurde mit einer Katalysatorbelastung von 0,16 kg / (kg Katalysator h) bei 90°C und einem Wasserstoffdruck von 100 bar an einem Kupferoxid-Aluminiumoxid-Katalysator (45/55; 3 mm Tabletten) in einem Rohrreaktor umgesetzt. Nach 900 Betriebsstunden unter konstanten Versuchsbedingungen beträgt der Umsatz 99 % bei einer Selektivität von 99 % (GC-Fl.%).

### Beispiel 2

Eine Lösung von 63 Gew.% 4-Chloracetophenon, 30 Gew.% Ethanol und 2 Gew.% Diethylamin wurde mit einer Katalysatorbelastung von 0,16 kg / (kg Katalysator h) bei 90°C und einem Wasserstoffdruck von 100 bar an einem Kupferoxid-Aluminiumoxid-Katalysator (45/55; 3 mm Tabletten) in einem Rohrreaktor umgesetzt. Nach 68 Betriebsstunden unter konstanten Versuchsbedingungen beträgt der Umsatz 98 % bei einer Selektivität von >99 % (GC-Fl.%).

### Beispiel 3

Wie Beispiel 2 jedoch mit n-Butylamin statt Diethylamin. Nach 20 Betriebsstunden unter konstanten Versuchsbedingungen beträgt der Umsatz 69 % bei einer Selektivität 80 % (GC-Fl.%).

### Beispiel 4

Eine Lösung von 62 Gew.% 4-Chloracetophenon, 25 Gew.% Methanol und 3 Gew.% Triethylamin wurde mit einer Katalysatorbelastung von 0,12 kg / (kg Katalysator h) bei 120°C und einem Wasserstoffdruck von 100 bar an einem Kupfer-Aluminiumoxid-Katalysator (45/55; 3 mm Tabletten) in einem Rohrreaktor umgesetzt. Nach 33 Betriebsstunden unter konstanten Versuchsbedingungen beträgt der Umsatz 85 % bei einer Selektivität 98 % (GC-Fl.%).

### Beispiel 5

Wie Beispiel 4 jedoch mit Tetrahydrofuran. Nach 24 Betriebsstunden unter konstanten Versuchsbedingungen beträgt 77 % beträgt die Selektivität 99 % (GC-Fl.%).

### Beispiel 6

Eine Lösung aus 75 Gew.% 4-Chloracetophenon und 25 Gew.% Ethanol wurde mit einer Katalysatorbelastung von 0,12 kg / (kg Katalysator h) bei 90°C und einem Wasserstoffdruck von 100 bar an einem Kupferoxid-Aluminiumoxid-Katalysator (45/55; 3 mm Tabletten) in einem Rohrreaktor umgesetzt. Nach 48 Betriebsstunden unter konstanten Versuchsbedingungen beträgt der Umsatz 54 % bei einer Selektivität >99 % (GC-Fl.%).

### Beispiel 7

Eine Lösung aus 97 Gew.% 4-Chloracetophenon und 3 Gew.% Triethylamin wurde mit einer Katalysatorbelastung von 0,12 kg / (kg Katalysator h) bei 120°C und einem Wasserstoffdruck von 100 bar an einem Kupferoxid-Aluminiumoxid-Katalysator (45/55; 3 mm Tabletten) in einem Rohrreaktor umgesetzt. Nach 39 Betriebsstunden unter konstanten Versuchsbedingungen beträgt der Umsatz 58 % bei einer Selektivität von >99 % (GC-FL.%).

### Beispiel 8

Eine Lösung aus 30 Gew.% 4-Chlorbenzaldehyd, 68 Gew.% Ethanol und 2 Gew.% Triethylamin wurde mit einer Katalysatorbelastung von 0,16 kg / (kg Katalysator h) bei 90°C und einem Wasserstoffdruck von 100 bar an einem Kupferoxid-Aluminiumoxid-Katalysator (45/55; 3 mm Tabletten) in einem Rohrreaktor umgesetzt. Nach 26 Betriebsstunden unter konstanten Versuchsbedingungen beträgt der Umsatz 97 % bei einer Selektivität >99 % (GC-Fl.%).

## Patentansprüche

1. Verfahren zur Herstellung von 4-Halogenbenzylalkoholen der allgemeinen Formel I, 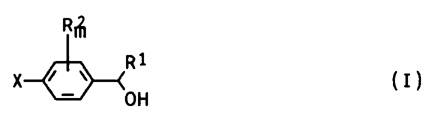 in der
R¹ Wasserstoff, C₁- bis C₂₀-Alkyl, C₂- bis C₂₀-Alkoxyalkyl, C₃- bis C₂₀-Cycloalkyl, C₄- bis C₃₀-Cycloalkylalkyl,
R² C₁- bis C₂₀-Alkyl, C₂- bis C₂₀-Alkoxyalkyl, C₃- bis C₂₀-Cycloalkyl, C₄- bis C₃₀-Cycloalkyl-alkyl,
X Halogen und
m 0 bis 2
bedeuten, dadurch gekennzeichnet, daß man halogenaromatische Carbonylverbindungen der Formel II 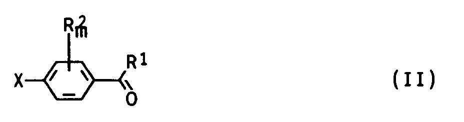 in der die Substituenten R¹, R² und X und der Index m die oben genannte Bedeutung haben, in Gegenwart eines Amins an Kupferkatalysatoren gegebenenfalls in Gegenwart eines inerten Lösungsmittel bei 50 bis 130°C und Drücken von 10 bis 200 bar, mit Wasserstoff umsetzt.

2. Verfahren zur Herstellung von 4-Halogenbenzylalkoholen der allgemeinen Formel I nach Anspruch 1, dadurch gekennzeichnet, daß man die Umsetzung bei Temperaturen von 70 bis 100°C und Drücken von 50 bis 150 bar durchführt.

3. Verfahren zur Herstellung von 4-Halogenbenzylalkoholen der allgemeinen Formel I nach Anspruch 1, dadurch gekennzeichnet, daß man die Umsetzung kontinuierlich durchführt.

4. Verfahren zur Herstellung von 4-Halogenbenzylalkoholen der allgemeinen Formel I nach Anspruch 1, dadurch gekennzeichnet, daß man als inerte Lösungsmittel C₁- bis C₂₀-Alkanole einsetzt.

5. Verfahren zur Herstellung von 4-Halogenbenzylalkoholen der allgemeinen Formel I nach Anspruch 1, dadurch gekennzeichnet, daß man als Amine aliphatische Amine der Formel NR³R⁴R⁵ einsetzt, in der R³, R⁴ und R⁵ Wasserstoff oder C₁- bis C₂₀-Alkyl bedeuten.
